# EUROPÄISCHE PATENTANMELDUNG

(11) **EP 1 585 049 A2**
(43) Veröffentlichungstag der Anmeldung: **12.10.2005**
(21) Anmeldenummer: 05090088.5
(22) Anmeldetag: 04.04.2005
(51) Int. Cl.: G06F 19/00

(54) **Verfahren und Vorrichtung zur Darstellung von digitalen Mammographieaufnahmen**

(30) Priorität: 05.04.2004 DE 102004017651
(71) Anmelder: Image Diagnost International GmbH, 81541 München (DE)
(72) Erfinder: Heinlein, Peter, Dr. rer. nat., 81549 München (DE); Forster, Brigitte, Dr. rer. nat., 81549 München (DE); Schneider, Wilfried, Dr.-Ing. habil., 85386 Eching (DE); Janssen, Siegfried, Dipl.-Phys., 85716 Unterschleissheim (DE)
(74) Vertreter: Müller, Wolfram Hubertus

(57) **Zusammenfassung**

Die Erfindung betrifft ein Verfahren zur Darstellung von digitalen Mammographieaufnahmen aus einem vorgespeicherten Datensatz. Dabei wird den Mammographieaufnahmen jeweils mindestens ein mammographiespezifisches Kennzeichen zugeordnet. Die Mammographieaufnahmen werden in einer Übersichtsdarstellung abhängig von ihrem jeweiligen mammographiespezifischen Kennzeichen automatisch vorsortiert angeordnet werden. Aus der Übersichtsdarstellung werden Mammographieaufnahmen ausgewählt und in einer Auswahldarstellung dargestellt. Weiterhin betrifft die Erfindung eine Vorrichtung zum Durchführen des Verfahrens. Die Erfindung dient dazu, eine verbesserte Darstellung von Mammographieaufnahmen zu ermöglichen und dadurch die Diagnose zu vereinfachen.

## Beschreibung

Die Erfindung betrifft ein Verfahren zur Darstellung digitaler Mammographieaufnahmen sowie eine Darstellungsvorrichtung dafür.

Unter Mammographie versteht man eine Röntgendarstellung der Brustdrüse, die üblicherweise mindestens zwei Mammographieaufnahmen der Brustdrüse aus unterschiedlichen Aufnahmerichtungen umfasst. Die Mammographie dient als technische Untersuchung zur Früherkennung von Karzinomen, bei der u.a. noch nicht ertastbare Tumore erkannt werden können. Bei einer solchen Vorsorgeuntersuchung werden in regelmäßigen Abständen neue Mammographieaufnahmen angefertigt.

Um eine manchmal im Verlaufe von Jahren auftretende Veränderung an den Aufnahmen feststellen zu können, werden die Mammographieaufnahmen digital gespeichert und können in so genannten Befundungsworkstations von Ärzten und/oder anderem Fachpersonal betrachtet und untersucht werden.

Der Erfindung liegt die Aufgabe zugrunde, eine verbesserte Darstellung von Mammographieaufnahmen zu ermöglichen.

Diese Aufgabe wird erfindungsgemäß durch ein Verfahren zur Darstellung digitaler Mammographieaufnahmen aus einem vorgespeicherten Datensatz gelöst. Dabei wird den Mammographieaufnahmen jeweils mindestens ein mammographiespezifisches Kennzeichen zugeordnet. Die zugeordneten mammographiespezifischen Kennzeichen werden in dem Datensatz mitgespeichert.

Die Darstellung der Mammographieaufnahmen erfolgt zum einen in einer Übersichtsdarstellung und zum anderen in einer Auswahldarstellung, in der nur aus der Übersichtsdarstellung ausgewählte Mammographieaufnahmen dargestellt werden. Die Mammographieaufnahmen werden in der Übersichtsdarstellung in Abhängigkeit von ihrem jeweiligen mammographiespezifischen Kennzeichen automatisch vorsortiert angeordnet.. Die Darstellung der Mammographieaufnahmen erfolgt also zweigeteilt: einmal werden beispielsweise alle Mammographieaufnahmen einer Patientin in der Übersichtsdarstellung dargestellt, zum anderen werden nur die daraus ausgewählten Mammographieaufnahmen in der Auswahldarstellung dargestellt.

Entscheidend bei der Anordnung in der Übersichtsdarstellung ist die automatische Vorsortierung. Da Mammographieaufnahmen standardisiert aus vorgegebenen Aufnahmeperspektiven aufgenommen werden, ist es durch die Vorsortierung möglich, die Aufnahmen in der Übersichtsdarstellung so anzuordnen, dass Veränderungen über die Zeit an einer bestimmten Stelle der Brustdrüse durch Vergleiche leicht erkannt werden können.

Bei der Vorsortierung wird also ausgenutzt, dass Mammographieaufnahmen aus standardisierten Aufnahmeperspektiven erfolgen. Die Sortierung erfolgt nach mammographiespezifischen Kennzeichen, beispielsweise nach Aufnahmerichtung, der Lateralität, dem Aufnahmewinkel, dem Vergrößerungsfaktor und/oder dem Patienten. Eine zusätzliche Sortierung kann auch nach dem Alter der Aufnahmen erfolgen, also dem Aufnahmedatum. In dem Fall wären die Mammographieaufnahmen nach steigendem oder abnehmendem Alter sortiert. Die Mammographieaufnahmen können insbesondere auch nach mehreren mammographiespezifischen Kennzeichen sortiert sein, wie beispielsweise Alter, Lateralität und Aufnahmerichtung zugleich.

Die Aufnahmerichtung von Mammographieaufnahmen wird üblicherweise unterschieden in "Craniocaudal", also eine Aufnahme in Kopf-Fuss-Richtung, und "Mediolateral Oblique" also eine Schrägaufnahme in seitlicher Darstellung. Mit Lateralität ist die Körperseite der Patientin gemeint, also entweder die rechte oder linke Brust.

Der vorgespeicherte Datensatz ist durch neu aufgenommene Mammographieaufnahmen erweiterbar, denen beim Abspeichern ihre mammographiespezifischen Kennzeichen zugeordnet werden.

Durch das erfindungsgemäße Verfahren wird es dem Arzt oder dem zuständigen Fachpersonal ermöglicht, schnell einen klaren Überblick über die vorhandenen Mammographieaufnahmen zu erhalten. Hat sich der Arzt bzw. das Fachpersonal erst einmal an die vorsortierte Anordnung in der Übersichtsdarstellung gewöhnt, kann er daraus schnell die für ihn wichtigen Mammographieaufnahmen auswählen, was wiederum eine vereinfachte Steuerung der Auswahl ermöglicht. Dadurch wird der Auswahlprozess vereinfacht und verkürzt, wobei zugleich die Diagnose verbessert wird.

In einer besonders bevorzugten Ausführungsform werden auch die aus der Übersichtsdarstellung ausgewählten Mammographieaufnahmen in der Übersichtsdarstellung abhängig von ihrem jeweiligen mammographiespezifischen Kennzeichen automatisch vorsortiert angeordnet. Die vorsortierte Anordnung ist dabei konfigurierbar, so dass sich der Arzt bzw. das Fachpersonal die Ordnung der Mammographieaufnahmen individuell und benutzerdefiniert konfigurieren kann. Die Sortierart ist abspeicherbar und auf alle unterschiedlichen Patienten übertragbar. Insbesondere können dabei in der Auswahldarstellung nicht nur fixe, vorsortierte Konstellationen von Mammographieaufnahmen dargestellt werden, sondern auch benutzerdefinierte Abfolgen von Mammographieaufnahmen. Diese werden unter anderem auch "Hanging protocols" genannt.

Dabei werden die ausgewählten Mammographieaufnahmen der Auswahldarstellung bevorzugt mittels eines Betätigungsmittels so gegen neu ausgewählte Mammographieaufnahmen ausgetauscht, dass sich an jeder Position der Auswahldarstellung die neu ausgewählte Mammographieaufnahme in genau einem mammographiespezifischen Kennzeichen von der zuvor an der Position angeordneten Mammographieaufnahme unterscheidet. Die übrigen mammographiespezifischen Kennzeichen der alten und neuen Mammographieaufnahme bleiben identisch. So wird eine Abfolge von Bildern bereitgestellt, die sich nur nach einem mammographiespezifischen Kennzeichen, wie beispielsweise dem Aufnahmedatum hin ändern. Dadurch kann beispielsweise die Zeitentwicklung einer bestimmten Aufnahme bzw. die Zeitentwicklung einer fixen Auswahl von Mammographieaufnahmen besonders gut mitverfolgt werden.

Vorteilhaft sind die Darstellungsausmaße der Mammographieaufnahmen in der Auswahldarstellung abhängig von der Anzahl der ausgewählten Mammographieaufnahmen. Wird die Anzahl der ausgewählten Mammographieaufnahmen verdoppelt, so halbiert sich der Platz, den eine Mammographieaufnahme bei maximaler Raumausnutzung des Darstellungsraums zur Verfügung hat. Die Darstellungsmaße der Mammographieaufnahmen werden also bezüglich des Darstellungsraumes in Abhängigkeit von der Anzahl der darzustellenden Mammographieaufnahmen in der Auswahldarstellung maximiert.

Zusätzlich ist es in einer Ausführungsform vorgesehen, dass einzelne Ausschnitte der Mammographieaufnahmen in einer Zoomansicht vergrößert dargestellt werden können. Durch ein Betätigungsmittel können so bestimmte Bereiche einer Aufnahme markiert und in der Zoomdarstellung dargestellt werden. Die Zoomdarstellung kann auch als einfache Variante der Auswahldarstellung ausgebildet sein. Dadurch können bestimmte Ausschnitte der Mammographieaufnahmen besonders gut und genau betrachtet werden.

In einer bevorzugten Ausführungsform werden die Mammographieaufnahmen auf einer Darstellungsfläche dargestellt, wobei die ausgewählten Mammographieaufnahmen der Auswahldarstellung als Hintergrund im Wesentlichen die ganze Darstellungsfläche bedecken. Die Mammographieaufnahmen der Übersichtsdarstellung werden zusammen mit den Mammographieaufnahmen der Auswahldarstellung auf der Darstellungsfläche dargestellt. Dabei sind die Mammographieaufnahmen der Übersichtsdarstellung im Vordergrund als eine Art Index angeordnet und bedecken nur einen Bruchteil der Darstellungsfläche. So können besonders effizient einzelne Bilder aus der vorsortierten Übersichtsanordnung in die Auswahldarstellung ausgewählt werden. Da die Mammographieaufnahmen in der Übersichtsanordnung vorsortiert sind, ist die Darstellung der Aufnahme in einem stark reduzierten Format in der Übersichtsdarstellung ausreichend, da der Arzt bzw. das Fachpersonal anhand der Position in der Übersichtsdarstellung erkennt, um welche Mammographieaufnahme es sich handelt. Aufgrund dieser Darstellung ergibt es sich, dass die Mammographieaufnahmen in der Auswahldarstellung gegenüber der Darstellung in der Übersichtsdarstellung vergrößert dargestellt werden können.

Die Aufgabe wird weiterhin von einer Darstellungsvorrichtung zur digitalen Darstellung von Mammographieaufnahmen eines vorgespeicherten Satzes gemäß dem vorstehend beschriebenen Verfahren gelöst. Danach weist die Darstellungsvorrichtung ein Sortiermittel auf, das die Mammographieaufnahmen nach mammographiespezifischen Kennzeichen automatisch vorsortiert und so vorsortiert in einer Übersichtsanordnung anordnet. Die Darstellungsvorrichtung umfasst weiterhin ein Darstellungsmittel, das die vom Sortiermittel in der Übersichtsanordnung vorsortierten Mammographieaufnahmen in einer Übersichtsdarstellung darstellt. Durch ein Betätigungsmittel sind einzelne Mammographieaufnahmen aus der Übersichtsanordnung auswählbar und in einer Auswahldarstellung durch das Darstellungsmittel darstellbar. Dabei sind Sortiermittel, Darstellungsmittel und Betätigungsmittel vorzugsweise standardmäßig hergestellte Bauteile bzw. Geräte. Die Herstellung spezieller Hardware zum Darstellen von Mammographieaufnahmen ist damit überflüssig.

Besonders bevorzugt umfasst das Betätigungsmittel einen Handheld-PC- mit einem Touchscreen. Dieser wird standardmäßig hergestellt und ist deswegen kostengünstig und wenig fehleranfällig, da seine Verwendung erprobt ist. Auf dem Touchscreen können in Abhängigkeit vom jeweiligen Betätigungsmodus verschiedene Tastenfunktionen konfiguriert sein. Dabei sind auf dem Touchscreen bevorzugt Mittel zum Markieren von Kontaktbereichen als Bedienungstasten ausgebildet. Dies kann beispielsweise durch eine Plastikfolie mit Kontakttasten oder eine Hülle mit Löchern an der Position der Tasten geschehen. Dadurch werden die Tasten fühlbar und der Arzt bzw. das Fachpersonal kann mit Hilfe des Betätigungsmittels, ohne von den dargestellten Mammographieaufnahmen wegzusehen, die dargestellten Bilder steuern.

Besonders bevorzugt umfasst das Betätigungsmittel eine standardisierte Cursorbewegungssteuerung wie beispielsweise ein Touchpad, einen Joystick, eine Maus oder beliebige Cursortasten. Den beiden Bewegungsrichtungen (rauf/runter und links/rechts) der Cursorbewegungssteuerung sind dabei mammographiespezifische Kennzeichen zugeordnet. Eine Betätigung der Cursorbewegungssteuerung in eine Bewegungsrichtung verändert dabei das mammographiespezifische Kennzeichen der in der Auswahldarstellung dargestellten Mammographieaufnahmen. Beispielsweise kann eine Bewegungsrichtung der Zeitachse zugeordnet sein, wodurch bei Betätigung des Betätigungsmittels die in der Auswahldarstellung dargestellten Mammographieaufnahmen entweder zu älteren oder jüngeren Mammographieaufnahmen wechseln. Die übrigen mammographiespezifischen Kennzeichen bleiben dabei konstant. Analog könnte anstatt des Aufnahmedatums auch die Aufnahmerichtung oder die Lateralität einer Bewegungsrichtung der Kursorbewegungssteuerung zugeordnet und steuerbar sein. Durch Wechseln in andere Betätigungsmodi können den Bewegungsrichtungen unterschiedliche mammographiespezifische Kennzeichen zugeordnet sein. Dabei kann in einem Betätigungsmodus der Cursorbewegungssteuerung auch die Helligkeit, der Kontrast, die Auflösung und/oder der Vergrößerungsfaktors der Mammographieaufnahme einstellbar sein. In einer Ausführungsform umfasst der Bildbetrachter zusätzlich zu den bereits beschriebenen Besonderheiten Funktionen eines standardmäßigen Bildbetrachtungsprogramms.

Die Erfindung wird nachfolgend anhand von in Figuren dargestellten Ausführungsformen näher erläutert. Es zeigen:
- Fig. 1: in einer schematischen Darstellung alle Mammographieaufnahmen einer Patientin automatisch in einer Übersichtsdarstellung vorsortiert;
- Fig. 2A bis 2D: verschiedene Belegungen von Steuertasten eines Betätigungsmittels zum Auswählen von Mammographieaufnahmen aus der Übersichtsanordnung und zum Bearbeiten der Bildanzeige von Mammographieaufnahmen in der Auswahldarstellung;
- Fig. 3: in schematischer Darstellung das Auswählen von Mammographieaufnahmen aus der Übersichtsanordnung der Figur 1 mit einem Steuermittel im Betätigungsmodus der Figur 2B;
- Fig. 4: in schematischer Darstellung ein zu dem in Figur 3 gezeigten alternatives Auswahlschema; und
- Fig. 5: in schematischer Darstellung das manuelle Auswählen einzelner Mammographieaufnahmen aus einer als Index im Vordergrund eines Darstellungsmittels angeordneten Übersichtsdarstellung.

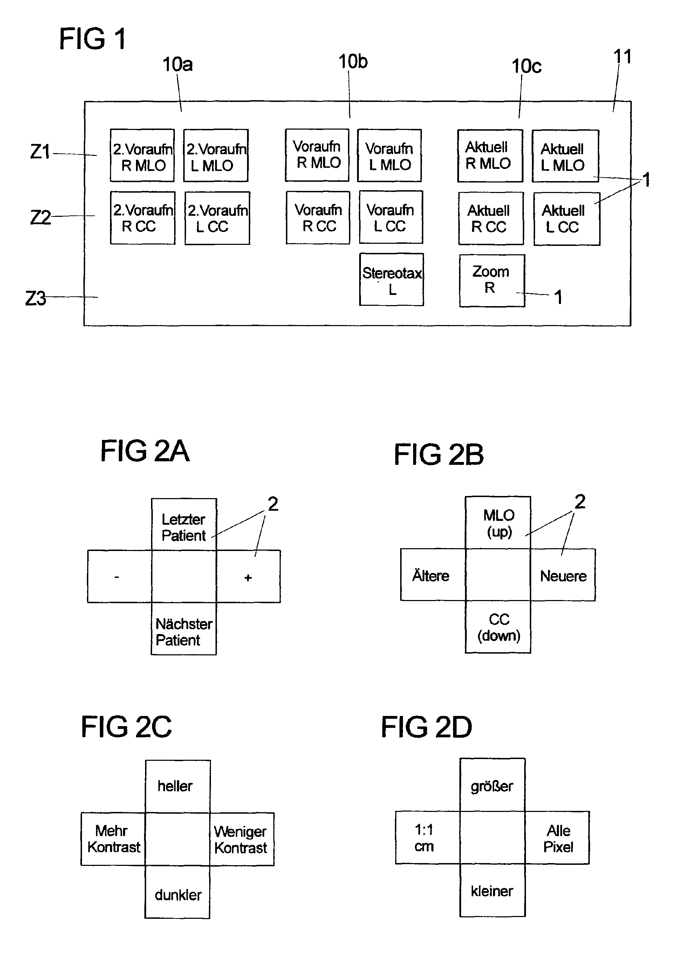

Die Figur 1 zeigt in einer schematischen Darstellung insgesamt vierzehn Mammographieaufnahmen 1 einer Patientin, die geordnet nach den mammographiespezifischen Kennzeichen der Mammographieaufnahmen in einer Übersichtsanordnung angeordnet und auf einem geeigneten Darstellungsmittel wie etwa einem Monitor in einer Übersichtsdarstellung 11 dargestellt sind.

Die Mammographieaufnahmen 1 sind in einem digitalen Bildformat auf einem nicht dargestellten Datenspeicher gespeichert. Als mammographiespezifisches Kennzeichen wird zu jeder Mammographieaufnahme 1 zusätzlich das Aufnahmedatum, die Lateralität und die Aufnahmerichtung als mammographiespezifisches Kennzeichen gespeichert. Die in Figur 1 quadratisch dargestellten Mammographieaufnahmen 1 sind zunächst in drei Spalten vorsortiert. In der rechten Spalte 10c sind fünf aktuelle Mammographieaufnahmen einer Patientin angeordnet. In der mittleren Spalte 10b sind fünf Mammographieaufnahmen 1 einer Voraufnahme angeordnet, die etwa zwei Jahre vor den aktuellen Mammographieaufnahmen aufgenommen wurden. In der linken Spalte 10a sind vier Mammographieaufnahmen 1 einer zweiten Voraufnahme angeordnet, die etwa vier Jahre alt sind. Das Alter der Mammographieaufnahmen 1 nimmt somit von Spalte zu Spalte und von links nach rechts in der Übersichtanordnung ab.

In der linken Spalte 10a sind die vier Mammographieaufnahmen 1 der zweiten Voraufnahme zunächst noch einmal in zwei Unterspalten aufgeteilt, einer linken und einer rechten. Die Mammographieaufnahmen 1 werden dabei je nach ihrer Lateralität der linken oder rechten Unterspalte der Spalte 10a zugeordnet. In der linken Unterspalte sind die Mammographieaufnahmen 1 der rechten Brust angeordnet (gekennzeichnet durch R), in der rechten Unterspalte sind die Mammographieaufnahmen 1 der linken Brust angeordnet (gekennzeichnet durch L). Diese Anordnung entspricht der Anordnung der Körperseiten einer Patientin, auf die ein Arzt frontal sieht.

Die Mammographieaufnahmen 1 der Spalte 10a sind weiterhin in zwei Zeilen aufgeteilt. In der ersten Zeile Z1 sind die Mammographieaufnahmen 1 mit der Aufnahmerichtung "Mediolateral Oblique" (MLO) angeordnet, während in der zweiten Zeile Z2 die Mammographieaufnahmen 1 mit der Aufnahmerichtung "Craniocaudal" (CC) angeordnet sind. Mammographieaufnahmen werden standardmäßig aus diesen beiden Perspektiven aufgenommen, so dass eine Zuordnung zu diesen Aufnahmerichtungen auch für ältere Mammographieaufnahmen in einem nichtdigitalisierten Format möglich ist, um einen vorgespeicherten Datensatz einer Patientin um ältere Aufnahmen zu erweitern.

In den beiden Spalten 10b und 10c sind die jeweils vier Mammographieaufnahmen 1 in den Zeilen Z1 und Z2 nach ihrer Lateralität und Aufnahmerichtung wie die vier Mammographieaufnahmen der Spalte 10a angeordnet. In der dritten Zeile Z3 ist sowohl in Spalte 10b als auch in Spalte 10c jeweils eine Zusatzaufnahme angeordnet. Diese Zusatzaufnahmen sind je nach Befund unterschiedlich und individuell auf die Patientin abgestimmt, beispielsweise als eine Vergrößerungsaufnahme oder als eine Stereotaxaufnahme. Soweit möglich, werden den Zusatzaufnahmen der Spalte Z3 ebenfalls mammographiespezifische Kennzeichen zugeordnet. Dies ist in jedem Fall möglich für die Lateralität und das Aufnahmedatum. Die Zusatzaufnahmen werden in Zeile Z3 je nach ihrem Aufnahmedatum zunächst ihrer Spalte (10a, 10b oder 10c) zugeordnet, und in dieser Spalte je nach Lateralität der richtigen Unterspalte.

Sind die Mammographieaufnahmen 1 mit samt ihren mammographiespezifischen Kennzeichen in dem Datensatz korrekt gespeichert, werden sie bei Abruf durch einen Benutzer automatisch von einem Sortiermittel wie beispielsweise einem Prozessor sortiert und ihrer richtigen Position in der Übersichtsanordnung zugeordnet. Die Darstellung erfolgt auf einem handelsüblichen Darstellungsmittel, wie einem Monitor.

In den Figuren 2A bis 2D sind vier unterschiedliche Betätigungsmodi eines nicht dargestellten Betätigungsmittels dargestellt, die dazu dienen, einzelne oder mehrere Mammographieaufnahmen aus einer Übersichtsdarstellung wie der in Figur 1 gezeigten Übersichtsdarstellung 11 auszuwählen und zu konfigurieren. Als Betätigungsmittel dient dabei ein Handheld-PC mit einem Touchscreen, auf dessen Touchscreen eine durchsichtige Plastikhülle vier kreuzförmig angeordnete Tasten 2 markiert. Die vier Tasten 2 liegen jeweils an einem Ende eines Kreuzes. Alle vier Betätigungsmodi der Figuren 2A und 2D können auf dem Handheld-PC aktiviert werden. Je nach aktiviertem Betätigungsmodus erscheint auf dem Touchscreen des Betätigungsmittels an der Position der vier Tasten 2 deren Wirkungsweise.

In Figur 2A wird durch Betätigung der oberen Taste "letzter Patient" bzw. der unteren Taste "nächster Patient" zwischen den Übersichtsdarstellungen einzelner Patientinnen gewechselt. Bei Betätigung werden alle Mammographieaufnahmen einer Patientin je nach Anzahl der Bilder nach dem Schema der Figur 1 in einer Übersichtsdarstellung 11 angeordnet. Mit den linken und rechten Tasten (plus und minus) können verschiedene "Hanging protocols" ausgewählt werden, also vordefinierten Bildabfolgen einer bestimmten Auswahl von Mammographieaufnahmen 1 einer Patientin.

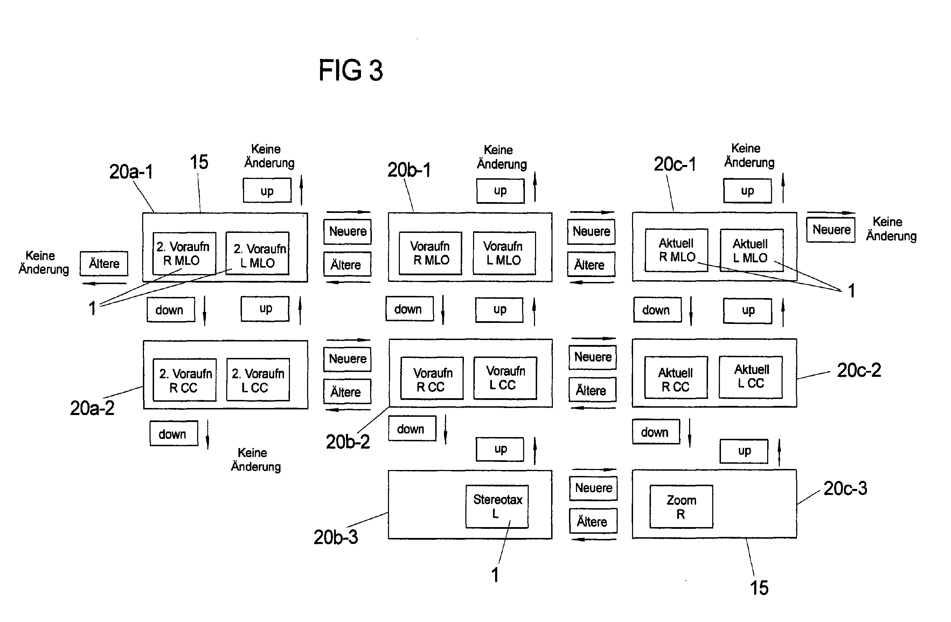

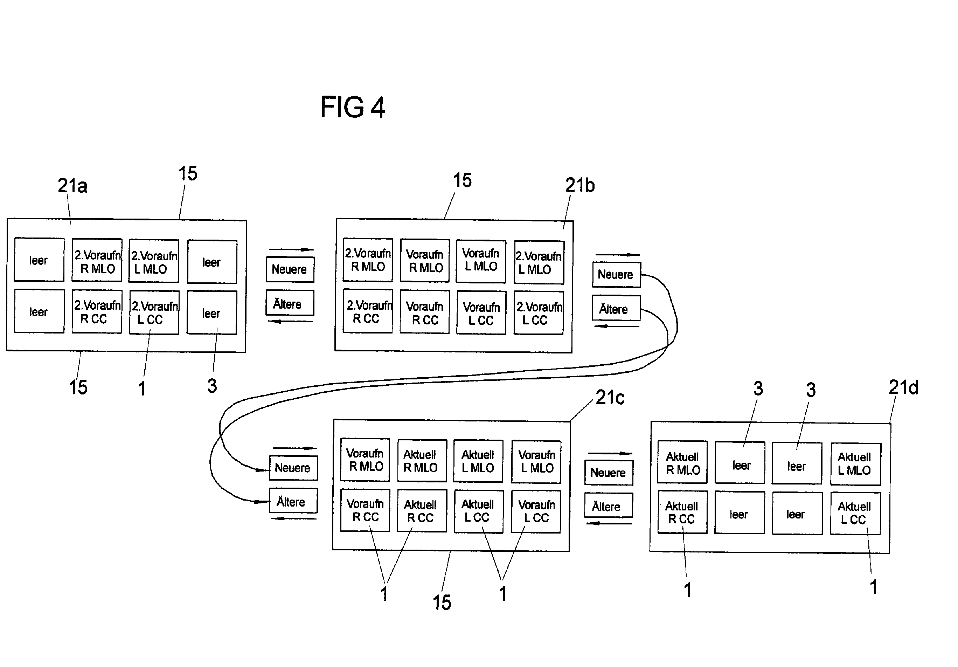

In der Figur 2B ist ein weiterer Betätigungsmodus des Betätigungsmittels gezeigt, der zum Auswählen von Mammographieausnahmen 1 aus der Übersichtsdarstellung 11 der Figur 1 dient. Jeder der Tasten 2 ist ein mammographiespezifisches Kennzeichen zugeordnet: Oben die MLO-Aufnahmerichtung, unten die CC-Aufnahmerichtung, sowie links die Funktion "ältere Aufnahme" und der rechten Taste die Funktion "neuere Aufnahme". Durch Betätigung einer der Tasten 2 wird an jeder Position einer Auswahldarstellung die jeweilige Mammographieaufnahme 1 durch eine Mammographieaufnahme ersetzt, die sich in genau dem mammographiespezifischen Kennzeichen von der ursprünglich angezeigten Mammographieaufnahme unterscheidet, das der betätigten Taste des Betätigungsmittels zugeordnet ist. Diese Funktionsweise wird näher in den Figuren 3 und 4 erläutert.

In den in den Figuren 2C und 2D dargestellten Betätigungsmodi können typische Bildeigenschaften wie Helligkeit, Kontrast und Auflösung durch die Tasten 2 des Betätigungsmittels eingestellt werden.

Bei dieser Art der Steuerung umfasst das Betätigungsmittel eine klassische Cursorbewegungssteuerung, also ein standardisiertes Gerät zum Bewegen des Cursors bzw. der Maus. Darunter fallen unter anderem eine Maus, ein Joystick, ein Touchpad, Cursortasten, einen Cyberhandschuh oder wie oben beschrieben ein Handheld-PC mit Touchscreen. Dabei wird zur einfacheren Orientierung für den Benutzer jede der beiden Bedienachsen (oben/unten und links/rechts) einem mammographiespezifischen Kennzeichen wie beispielsweise die Aufnahmerichtung oder das Aufnahmedatum zugeordnet, oder aber eine klassische Bildbearbeitungsfunktion wie Helligkeitseinstellung etc..

Die Figur 3 zeigt diverse Auswahldarstellungen 20a-1 bis 20c-3 und das Auswählen von Mammographieaufnahmen 1 in diese Auswahldarstellungen 20a-1 bis 20c-3 durch ein Betätigungsmittel im Betätigungsmodus der Figur 2B. Im in der Figur 3 gezeigten Beispiel besteht eine Auswahldarstellung aus zwei Mammographieaufnahmen 1, die jeweils in der linken bzw. rechten Hälfte eines Monitors angeordnet sind. Die Auswahldarstellung 20a-1 zeigt die Aufnahmen der linken und rechten Brust einer Patientin in der zweiten Voraufnahme für die MLO-Aufnahmerichtung. Die Betätigung der Taste "neuer" tauscht die beiden Mammographieaufnahmen 1 der Auswahldarstellung 20a-1 gegen die Mammographieaufnahmen 1 der ersten Voraufnahme mit gleicher Lateralität und Aufnahmerichtung aus. Diese sind in der Auswahldarstellung 20a-2 dargestellt. Durch Betätigung der Taste "down" (vgl. Figur 2B) werden die Mammographieaufnahmen 1 der Auswahldarstellung 20a-2 gegen Mammographieaufnahmen 1 mit demselben Aufnahmedatum und derselben Lateralität, aber mit unterschiedlicher Aufnahmerichtung ausgewechselt. Das Darstellungsmittel zeigt dabei die Auswahldarstellung 20b-2. Auf diese Art kann der Benutzer durch das Betätigungsmittel sehr einfach gewünschte Mammographieaufnahmen aus der Übersichtsdarstellung mit allen Aufnahmen von einer Patientin auswählen und in der Auswahldarstellung 20a-1 bis 20c-3 darstellen. Durch dieses Verfahren zur Auswahl von Mammographieausnahmen aus allen vorhandenen Mammographieaufnahmen einer Patientin ist gegenüber dem Stand der Technik deutlich vereinfacht und beschleunigt.

Die Figur 3 zeigt also, wie durch das Betätigen einer Taste 2 des Betätigungselements Mammographieaufnahmen 1 nach ihren ausgewählten mammographiespezifischen Kennzeichen aus der Übersichtsdarstellung ausgewählt und in der Auswahldarstellung dargestellt werden. Beim Betätigen von Tasten 2 des Betätigungsmittels ausgehend von einer beliebigen Auswahldarstellung 20a-1 bis 20c-3 ist die sich ergebende Auswahldarstellung der Figur 3 zu entnehmen.

In Figur 4 wird eine alternative Aufteilung des Darstellungsraumes auf dem Darstellungsmittel 15 gezeigt. In der Auswahldarstellung ist der Darstellungsraum des Darstellungsmittels 15, also beispielsweise der Bildschirm eines Monitors, in insgesamt acht gleichgroße Flächen für jeweils eine Mammographieaufnahme 1 aufgeteilt. Jede der Auswahldarstellungen 21a bis 21d zeigt bei diesem Betrachtungsmodus die vier Mammographieaufnahmen 1 eines bestimmten, ausgewählten Aufnahmedatums in der Mitte des Darstellungsmittels 15, während neben jeder Mammographieaufnahme 1 am Rand des Darstellungsmittels 15 die jeweils bei der zeitlich älteren Aufnahme gemachte Mammographieaufnahme 1 mit derselben Lateralität und derselben Aufnahmerichtung angeordnet ist. Mit einem Betätigungsmittel kann das Alter der vier zentral in der Auswahldarstellung angeordneten Mammographieaufnahmen 1 geändert werden, also von einer der Auswahldarstellungen 21a bis 21d in eine benachbarte wechseln. Positionen auf dem Darstellungsmittel 15, die nicht von Mammographieaufnahmen 1 besetzt sind, werden von einer leeren Fläche als Platzhalter 3 bedeckt.

Alternativ kann bei dem Verfahren ein "Hanging protocol" so definiert werden, dass die Bildabfolge 21a bis 21d oder umgekehrt automatisch als Auswahldarstellung mit einem bestimmbaren Darstellungszeitraum dargestellt wird.

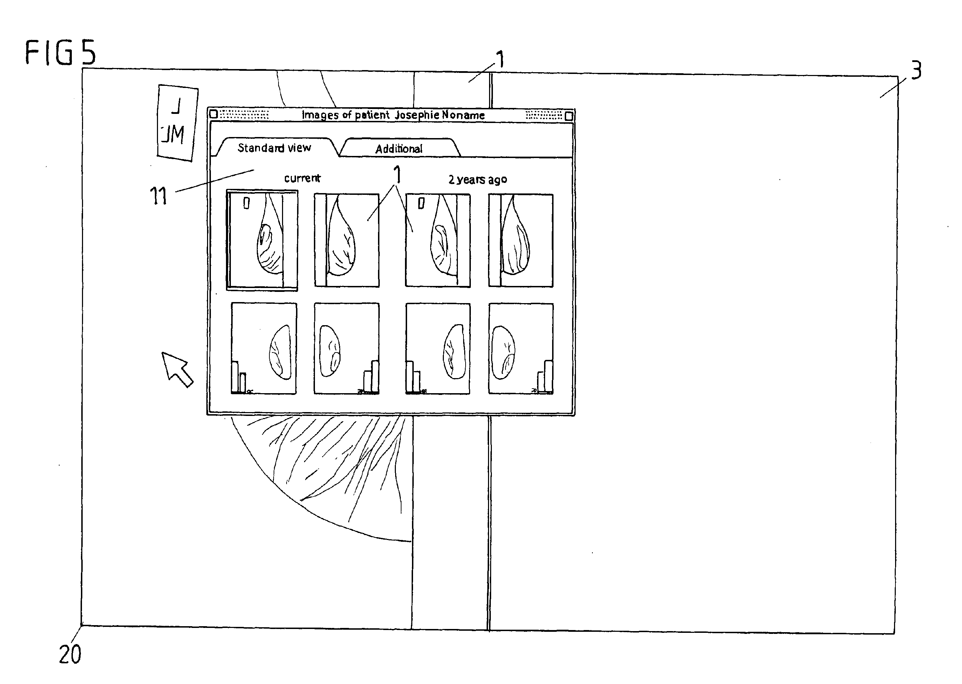

In Figur 5 ist ein Monitor als Darstellungsmittel 15 gezeigt, in dessen Vordergrund als Index alle Mammographieaufnahmen 1 einer Patientin vorsortiert angeordnet sind. Die Übersichtsdarstellung überdeckt dabei etwa ein Viertel des Monitors 15. Einzelne Mammographieaufnahmen 1 können aus der Übersichtsdarstellung 11 ausgewählt und mittels "drag and drop" oder Markieren bzw. durch einen "Doppelklick" ausgewählt und in die Auswahldarstellung 20 ausgewählt werden. Ist eine bestimmte Anzahl von Mammographieaufnahmen 1 (beispielsweise eine, zwei, vier oder acht - je nach gewünschter Aufteilung des Monitors 15) in die Auswahldarstellung 20 ausgewählt worden, so wird die Auswahldarstellung 20 in den Vordergrund geschaltet und die ausgewählten Mammographieaufnahmen 1 durch ein Betätigungsmittel im Betätigungsmodus der Figur 2B verändert bzw. gegen andere Mammographieaufnahmen 1 der Patientin ausgetauscht.

## Patentansprüche

1. Verfahren zur Darstellung digitaler Mammographieaufnahmen (1) aus einem vorgespeicherten Datensatz, wobei
- den Mammographieaufnahmen (1) jeweils mindestens ein mammographiespezifisches Kennzeichen zugeordnet wird,
- die Mammographieaufnahmen (1) in einer Übersichtsdarstellung (11) abhängig von ihrem jeweiligen mammographiespezifischen Kennzeichen automatisch vorsortiert angeordnet werden und
- aus der Übersichtsdarstellung (11) Mammographieaufnahmen (1) ausgewählt und zusätzlich in einer Auswahldarstellung (20; 20a-1 bis 20c-3; 21 a bis 21 d) dargestellt werden.

2. Verfahren nach Anspruch 1, **dadurch gekennzeichnet, dass** den Mammographieaufnahmen (1) jeweils ihre Aufnahmerichtung, ihre Lateralität, ihr Vergrößerungsfaktor, ihr Aufnahmedatum und/oder die von ihr aufgenommene Patientin als mammographiespezifisches Kennzeichen zugeordnet wird.

3. Verfahren nach einem der vorangegangenen Ansprüche, **dadurch gekennzeichnet, dass** in der Übersichtsdarstellung (11) alle Mammographieaufnahmen (1) einer Patientin dargestellt werden.

4. Verfahren nach einem der vorangegangenen Ansprüche, **dadurch gekennzeichnet, dass** Mammographieaufnahmen (1) nach ihren mammographiespezifischen Kennzeichen aus der Übersichtsdarstellung (11) in die Auswahldarstellung (20; 20a-1 bis 20c-3; 21 a bis 21 d) ausgewählt werden.

5. Verfahren nach einem der vorangegangenen Ansprüche, **dadurch gekennzeichnet, dass** die ausgewählten Mammographieaufnahmen (1) in der Auswahldarstellung (20; 20a-1 bis 20c-3; 21a bis 21d) abhängig von ihrem jeweiligen mammographiespezifischen Kennzeichen automatisch vorsortiert angeordnet werden.

6. Verfahren nach Anspruch 4 oder 5, **dadurch gekennzeichnet, dass** die ausgewählten Mammographieaufnahmen (1) der Auswahldarstellung (20; 20a-1 bis 20c-3; 21a bis 21d) mittels eines Betätigungsmittels so gegen neu ausgewählte Mammographieaufnahmen (1) ausgetauscht werden, dass sich an jeder Position der Auswahldarstellung (20; 20a-1 bis 20c-3; 21a bis 21d) die neu ausgewählte Mammographieaufnahme (1) durch genau ein mammographiespezifisches Kennzeichen von der zuvor an der Position angeordneten Mammographieaufnahme (1) unterscheidet, während die übrigen mammographiespezifischen Kennzeichen einander entsprechen.

7. Verfahren nach einem der vorangegangenen Ansprüche, **dadurch gekennzeichnet, dass** die Darstellungsausmaße der Mammographieaufnahmen (1) in der Auswahldarstellung (20; 20a-1 bis 20c-3; 21a bis 21d) abhängig von der Anzahl der ausgewählten Mammographieaufnahmen (1) sind.

8. Verfahren nach einem der vorangegangenen Ansprüche, **dadurch gekennzeichnet, dass** Ausschnitte der Mammographieaufnahmen (1) in einer Zoomansicht vergrößert dargestellt werden.

9. Verfahren nach einem der vorangegangenen Ansprüche, **dadurch gekennzeichnet, dass** die Mammographieaufnahmen (1) auf einer Darstellungsfläche dargestellt werden, wobei die Auswahldarstellung (20; 20a-1 bis 20c-3; 21 a bis 21d) als Hintergrund im Wesentlichen die ganze Darstellungsfläche bedeckt, und wobei die Übersichtsdarstellung (11) als Index im Vordergrund der Darstellungsfläche nur einen Bruchteil der Darstellungsfläche bedeckt.

10. Verfahren nach einem der vorangegangenen Ansprüche, **dadurch gekennzeichnet, dass** die Mammographieaufnahmen (1) in der Auswahldarstellung (20; 20a-1 bis 20c-3; 21 a bis 21d) vergrößert gegenüber der Darstellung in der Übersichtsdarstellung (11) dargestellt werden.

11. Verfahren nach einem der vorangegangenen Ansprüche, **dadurch gekennzeichnet, dass** die in der Auswahldarstellung (20; 20a-1 bis 20c-3; 21a bis 21d) dargestellten Mammographieaufnahmen (1) in von ihren mammographiespezifischen Kennzeichen abhängigen und vordefinierten Bildabfolgen automatisch dargestellt werden.

12. Darstellungsvorrichtung zur Darstellung digitaler Mammographieaufnahmen (1) eines vorgespeicherten Datensatzes gemäß einem Verfahren nach einem der vorangegangenen Ansprüche, mit
- einem Sortiermittel, das die Mammographieaufnahmen (1) nach mammo- graphiespezifischen Kennzeichen automatisch vorsortiert und vorsortiert in einer Übersichtsanordnung anordnet;
- einem Darstellungsmittel (15), das die vom Sortiermittel in der Übersichts- anordnung vorsortierten Mammographieaufnahmen (1) in einer Über- sichtsdarstellung (11) darstellt;
- Betätigungsmittel, durch das die Mammographieaufnahmen (1) aus der Übersichtsdarstellung (11) auswählbar und in einer Auswahldarstellung (20; 20a-1 bis 20c-3; 21 a bis 21 d) durch das Darstellungsmittel (15) dar- stellbar sind.

13. Darstellungsvorrichtung nach einem der Ansprüche 12, **dadurch gekennzeichnet, dass** das Betätigungsmittel eine standardisierte Cursorbewegungssteuerung umfasst, wobei die Bewegungsrichtungen der Cursorbewegungssteuerung einem mammographiespezifischen Kennzeichen zugeordnet sind.

14. Darstellungsvorrichtung nach Anspruch 13, **dadurch gekennzeichnet, dass** durch Betätigung der Cursorbewegungssteuerung die ausgewählten Mammographieaufnahmen (1) der Auswahldarstellung (20; 20a-1 bis 20c-3; 21a bis 21d) so gegen neu ausgewählte Mammographieaufnahmen (1) austauschbar sind, dass sich an jeder Position der Auswahldarstellung (20; 20a-1 bis 20c-3; 21a bis 21d) die neu ausgewählte Mammographieaufnahme (1) durch genau ein mammographiespezifisches Kennzeichen von der zuvor an der Position angeordneten Mammographieaufnahme (1) unterscheidet, während die übrigen mammographiespezifischen Kennzeichen einander entsprechen.

15. Darstellungsvorrichtung nach Anspruch 13 oder 14, **dadurch gekennzeichnet, dass** durch Betätigung der Cursorbewegungssteuerung die Helligkeit, der Kontrast, die Auflösung und/oder der Vergrößerungsfaktor mindestens einer auf dem Darstellungsmittel dargestellten Mammographieaufnahme (1) einstellbar ist.

16. Darstellungsvorrichtung nach einem der Ansprüche 12 bis 15, **dadurch gekennzeichnet, dass** das Betätigungsmittel einen Handheld-PC mit Touchscreen umfasst.

17. Darstellungsvorrichtung nach Anspruch 16, **dadurch gekennzeichnet, dass** auf dem Touchscreen Mittel zum Markieren von Kontaktbereichen als Bedienungstasten ausgebildet ist.
